(19) Europäisches Patentamt
European Patent Office
Office européen des brevets

(11) Veröffentlichungsnummer: **0 233 173**
**A1**

(12) # EUROPÄISCHE PATENTANMELDUNG

(21) Anmeldenummer: **87890016.6**

(22) Anmeldetag: **28.01.87**

(51) Int. Cl.4: **C 07 D 333/32**
A 61 K 31/38

(30) Priorität: **29.01.86 AT 212/86**

(43) Veröffentlichungstag der Anmeldung:
**19.08.87 Patentblatt 87/34**

(84) Benannte Vertragsstaaten:
**AT BE CH DE ES FR GB IT LI LU NL SE**

(71) Anmelder: **Laevosan-Gesellschaft m.b.H.**
**Estermannstrasse 17**
**A-4020 Linz (AT)**

(72) Erfinder: **Binder, Dieter, Dr.**
**Sieveringerstrasse 207**
**A-1190 Wien (AT)**

**Ferber, Hubert, Dr.**
**Ahornweg 24**
**A-4052 Ansfelden (AT)**

(74) Vertreter: **Pawloy, Heinrich, Dr. et al**
**Riemergasse 14**
**A-1010 Wien (AT)**

Patentansprüche für folgenden Vertragsstaten: AT + ES.

(54) **1-[3-(2-Hydroxy-3-alkylaminopropoxy)-2-thienyl]-3-phenyl-1-propanone und ihre Säureadditionssalze sowie Verfahren zu ihrer Herstellung und sie enthaltende pharmazeutische Zusammensetzungen.**

(57) 1-[3-(2-Hydroxy-3-alkylaminopropoxy)-2-thienyl]-3-phenyl-1-propanone der allgemeinen Formel

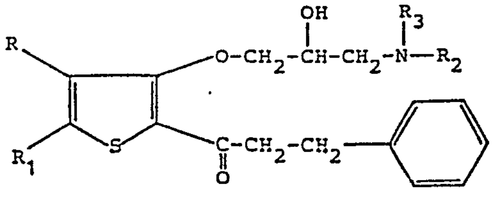

(I),

worin R und $R_1$ jeweils Wasserstoff oder Methyl bedeuten, wobei aber mindestens eines hievon Methyl bedeutet, $R_2$ Alkyl, Cycloalkl oder Alkenyl mit jeweils bis zu 8 C-Atomen bedeutet und $R_3$ Wasserstoff darstellt oder $R_2$ und $R_3$ zusammen mit dem sie verbindenden Stickstoffatom einen 6-gliedrigen gesättigten heterocyclischen Ring bilden, der gegebenenfalls durch einen Alkylrest mit 1 bis 3 C-Atomen substituiert sein kann, mit der Maßgabe, daß $R_2$ eine andere Bedeutung als Propyl, n-Butyl, Isobutyl oder tert.Butyl hat, wenn $R_3$ Wasserstoff ist, und ihre Säureadditionssalze sind zur Behandlung von Herzrhythmusstörungen verwendbar.

EP 0 233 173 A1

**Beschreibung**

1-[3-(2-Hydroxy-3-alkylaminopropoxy)-2-thienyl]-3-phenyl-1-propanone und ihre Säureadditionssalze sowie Verfahren zu ihrer Herstellung und sie enthaltende pharmazeutische Zusammensetzungen

Die Erfindung betrifft neue therapeutisch wertvolle Derivate des 1-[3-(2-Hydroxy-3-alkylaminopropoxy)-2-thienyl]-3-phenyl-1-propanons der allgemeinen Formel

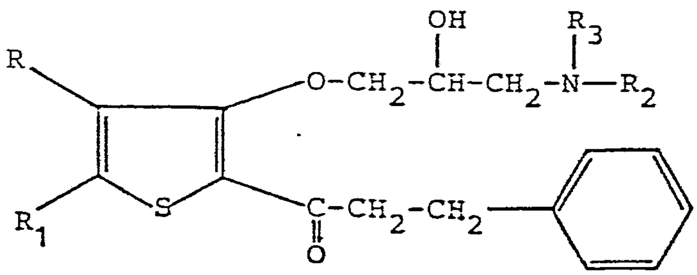

(I),

worin R und $R_1$ jeweils Wasserstoff oder Methyl bedeuten, wobei aber mindestens eines hievon Methyl bedeutet, $R_2$ Alkyl, Cycloalkyl oder Alkenyl mit jeweils bis zu 8 C-Atomen bedeutet und $R_3$ Wasserstoff darstellt oder $R_2$ und $R_3$ zusammen mit dem sie verbindenden Stickstoffatom einen 6-gliedrigen gesättigten heterocyclischen Ring bilden, der gegebenenfalls durch einen Alkylrest mit 1 bis 3 C-Atomen substituiert sein kann, mit der Maßgabe, daß $R_2$ eine andere Bedeutung als Propyl, n-Butyl, Isobutyl oder tert.Butyl hat, wenn $R_3$ Wasserstoff ist, und ihre Säureadditionssalze.

Aus der AT-PS 369 739 sind bereits Verbindungen der Formel (I) bekannt, worin R und $R_1$ die obige Bedeutung haben, $R_3$ Wasserstoff ist und $R_2$ n-Propyl, n-Butyl, Isobutyl oder tert.-Butyl bedeutet. Es hat sich nun überraschenderweise herausgestellt, daß $R_2$ und $R_3$ weitgehend variiert werden können, ohne daß dabei die gewünschte Wirkung der Verbindungen negativ beeinflußt wird. Es ist sogar möglich, durch Variation von $R_2$ und $R_3$ Verbindungen zu erhalten, welche in bestimmter Hin sicht den bisher bekannten Verbindungen überlegen sind. Die erfindungsgemäßen Verbindungen besitzen hervorragende antiarrhythmische Wirksamkeit, insbesondere bei oraler Verabreichung. Die Variationsmöglichkeiten an $R_2$ und $R_3$ sind insbesondere deshalb von wesentlicher Bedeutung, als es dadurch möglich ist, eventuelle unerwünschte Nebenwirkungen leichter zu vermeiden und Verbindungen herzustellen, welche dem jeweiligen Krankheitsbild besser angepaßt werden können.

Aus der DE-OS 33 16 155 ist 2-[2'-Hydroxy-3'-(1,1-dimethylpropylamino)-propoxy]-1-thienylpropiophenon bekannt, eine Verbindung mit guter antiarrhythmischer Wirksamkeit.

Aufgrund der obgenannten pharmakologischen Eigenschaften können die erfindungsgemäßen Verbindungen oder ihre Säureadditionssalze allein oder in Mischung mit anderen Wirksubstanzen in Form üblicher galenischer Zubereitungen (auch Retardformen) bei Herzrhythmusstörungen als Medikament angewendet werden.

Die Erfindung bezieht sich weiterhin auf ein Verfahren zur Herstellung der Verbindungen der Formel (I).

Das erfindungsgemäße Verfahren ist dadurch gekennzeichnet, daß man eine Verbindung der allgemeinen Formel

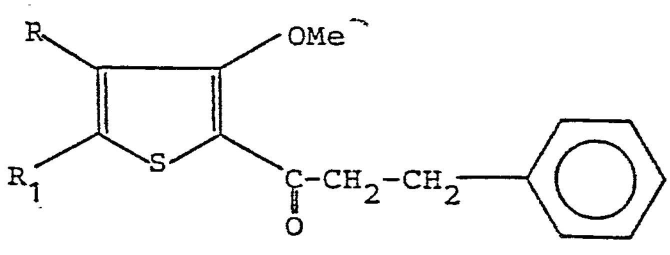

(II),

worin R und $R_1$ die oben genannte Bedeutung besitzen, und Me ein Alkalimetall-Kation bedeutet, mit Epichlorhydrin der Formel

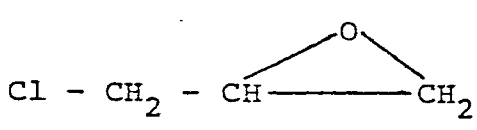

(III)

in einem reaktionsinerten Lösungsmittel oder besser in überschüssigem Epichlorhydrin bei Temperaturen zwischen 110 und 140°C umsetzt und die so erhaltene Verbindung der Formel

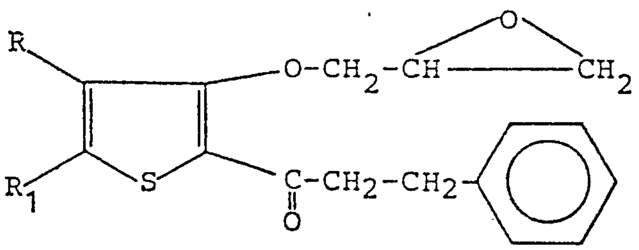

(IV) ,

worin R und $R_1$ die oben genannte Bedeutung besitzen, mit einem Alkylamin der allgemeinen Formel

$R_2R_3$ - NH (V)

worin $R_2$ und $R_3$ die oben genannte Bedeutung besitzen, in einem reaktionsinerten Lösungsmittel oder besser in überschüssigem Amin der Formel (V) bei einer Temperatur zwischen 50 und 100°C umsetzt und die erhaltenen Basen der Formel (I) gegebenenfalls in die Säureadditionssalze überführt.

Die Säureadditionssalze der Endstoffe können in an sich bekannter Weise z.B. mit Alkalien oder Ionenaustauschern in die freien Basen überführt werden, von denen sich durch Umsetzung mit anorganischen oder organischen Säuren, insbesondere solchen, die zur Bildung von therapeutisch verwendbaren Salzen geeignet sind, weitere Salze gewinnen lassen.

Infolge der engen Beziehungen zwischen den neuen Verbindungen und deren Salzen sind im vorausgegangenen und nachfolgenden sinn- und zweckmäßig gegebenenfalls auch unter den freien Basen die entsprechenden Salze zu verstehen.

Die Ausgangsmaterialien der Formel (II), (III) und (V) sind literaturbekannt (siehe z.B. AT-PS 369 739).

Die folgenden Beispiele sollen die vorliegende Erfindung näher erläutern, ohne daß diese hierauf beschränkt sein soll.

Zunächst wird die Herstellung einer Ausgangsverbindung der Formel (IV) erläutert:

1-[3-(2,3-Epoxypropoxy)-4-methyl-2-thienyl]-3-phenyl-1-propanon (Formel (IV): R = $CH_3$, $R_1$ = H)

1,74 g (0,076 Mol) Na werden in 50 ml abs. Methanol gelöst, 18,84 g (0,076 Mol) 1-(3-Hydroxy-4-methyl-2-thienyl)-3-phenyl-1-propanon (Formel II), R = $CH_3$, $R_1$ = H, Me = H) eingetragen und die Lösung zur Trockene (im Vakuum) eingedampft. Der kristalline Rückstand (Formel (II), R = $CH_3$, $R_1$ = H, Me = Na) wird mit 50 ml Epichlorhydrin (Formel III)) versetzt und 4 h am Rückfluß erhitzt. Die Reaktionsmischung wird über ein Filterhilfsmittel aus hochgereinigter Diatomeenerde (Hyflo) abgesaugt, mit etwas Benzol nachgespült und das Filtrat im Vakuum eingedampft. Der kristalline Rückstand wird in 1250 ml Cyclohexan unter Zugabe von Aktivkohle aufgekocht, über Hyflo abgesaugt und das Filtrat im Vakuum eingedampft. Der ölige Rückstand wiegt 16,9 g (77,6 %). Das Produkt ist für den weiteren Einsatz rein genug.

Analog kann man erhalten:

1-[3-(2,3-Epoxypropoxy)-5-methyl-2-thienyl]-3-phenyl-1-propanon (Formel (IV), R = H, $R_1$ = $CH_3$), Öl, nicht isoliert.

1-[4,5-Dimethyl-3-(2,3-epoxypropoxy)-2-thienyl]-3-phenyl-1-propanon (Formel(IV), R und $R_1$ = $CH_3$) Öl, nicht isoliert, $n_D^{20}$ : 1,5535.

**Beispiel 1:** 1-[4-Methyl-3-(2-hydroxy-3-sek.butylaminopropoxy)-2-thienyl]-3-phenyl-1-propanon-chlorhydrat (Formel (I).HCl, R = $CH_3$, $R_1$ und $R_2$ = H, $R_3$ = sek.Butyl).

15,0 g (0,05 Mol) rohes 1-[3-(2,3-Epoxypropoxy)-4-methyl-2-thienyl]-3-phenyl-1-propanon (Formel (IV), R = $CH_3$, $R_1$ = H) werden in 60 ml sek.Butylamin (Formel (V), $R_2$ = H, $R_3$ = sek.-Butyl) 4 h unter Rühren am Rückfluß erhitzt. Die Reaktionsmischung wird im Vakuum eingedampft, der Rückstand zwischen 250 ml $CH_2Cl_2$ und 150 ml 1n HCl verteilt, die Phasen werden getrennt, die wässerige Phase noch zweimal mit je 50 ml $CH_2Cl_2$ extrahiert und die vereinigten organischen Phasen werden getrocknet ($Na_2SO_4$) und im Vakuum eingedampft. Das ölige Rohprodukt kristallisiert bei Anreiben mit Aceton und wird daraus mit Aktivkohle umkristallisiert. Ausbeute 5,9 g (29 %) farblose Kristalle, Fp. 108 - 111°C, MG = 411,987.

**Beispiel 2:**

1-[3-(2-Hydroxy-3-tert.pentylaminopropoxy)-4-methyl-2-thienyl]-3-phenyl-1-propanon-hydrochlorid

10 g Epoxid werden mit 60 ml tert.Pentylamin 5 h lang unter Rühren am Rückfluß gekocht (KOH-Trockenrohr). Dann wird das überschüssige Amin am Rotationsverdampfer abdestilliert und das zurückbleibende konstant getrocknete Öl in 50 ml Methylenchlorid gelöst und mit 60 ml 4n HCl intensiv geschüttelt.

Die wässerige Phase wird noch fünfmal mit je 60 ml Methylenchlorid extrahiert, die vereinigten organischen Phasen werden unter Zusatz von 3 g Aktivkohle über $Na_2SO_4$ getrocknet, eingedampft und das zurückbleibende Öl bis zu Gewichtskonstanz getrocknet.

Das Rohprodukt wird über eine Kieselgelsäule gereinigt. Die Verunreinigungen werden mit Toluol/Äther (3/1) eluiert und das Umsetzungsprodukt mit Äthanol eluiert. Die äthanolische Lösung wird eingedampft, und

der Rückstand aus 40 ml Aceton p.A. umkristallisiert.

Ausbeute: 4,6 g (33 %), Fp. 109 - 112°C; farblose Kristalle, MG = 426,014.

**Beispiel 3:**

1-[3-(2-Hydroxy-3-(2,2-dimethylpropylamino)-propoxy)-4-methyl-2-thienyl]-3-phenyl-1-propanon-hydrochlorid

3,0 g Epoxid werden mit 20 ml 2,2-Dimethylpropylamin 5 h lang unter Rühren am Rückfluß gekocht (KOH-Trockenrohr). Dann wird das überschüssige Amin am Rotationsverdampfer abdestilliert und das zurückbleibende getrocknete Öl in 10 ml Methylenchlorid gelöst und mit 15 ml 4n HCl intensiv geschüttelt. Die wässerige Phase wird noch fünfmal mit je 10 ml Methylenchlorid extrahiert, die vereinten organischen Phasen werden mit $Na_2SO_4$ getrocknet und das zurückbleibende Öl aus 5 ml Aceton umkristallisiert.

Ausbeute: 1,16 g (27 %), Fp 135 - 138°C; farblose Kristalle, MG = 426,014.

**Beispiel 4:**

1-[3-(2-Hydroxy-3-(4-methyl)-1-piperidinyl)-propoxy)-4-methyl-2-thienyl]-3-phenyl-1-propanon-hydrochlorid

2,0 g Epoxid werden mit 15 ml 4-Methylpiperidin 5 h bei 49 - 50°C gerührt (KOH-Trockenrohr). Nach dem Abkühlen wird das überschüssige 4-Methylpiperidin im Vakuum abdestilliert und der getrocknete Rückstand in 20 ml Methylenchlorid gelöst und mit 15 ml 4n HCl intensiv geschüttelt, die wässerige Phase noch fünfmal mit je 10 ml Methylenchlorid extrahiert, die organische Phase mit $Na_2SO_4$ und Aktivkohle behandelt, zweimal filtriert und eingedampft. Das zurückbleibende Öl wird bis zur Gewichtskonstanz getrocknet und der Rückstand in 5 ml Aceton umkristallisiert.

Ausbeute: 1,59 g (55 %), Fp. 162 - 165°C; fast farblose Kristalle, MG = 438,03.

**Beispiel 5:**

1-[3-(2-Hydroxy-3-(2-methyl-1-butylamino)-propoxy)-4-methyl-2-thienyl]-3-phenyl-1-propanon-hydrochlorid

3,0 g Epoxid werden mit 20 ml 2-Methyl-1-butylamin 5 h lang unter Rückfluß gekocht (KOH-Trockenrohr). Dann wird das überschüssige Amin am Rotationsverdampfer abdestilliert und der getrocknete Rückstand in 20 ml Methylenchlorid gelöst und mit 15 ml 4n HCl intensiv geschüttelt. Die wässerige Phase wird noch fünfmal mit je 10 ml Methylenchlorid extrahiert; die organischen Phasen werden mit $Na_2SO_4$ und Aktivkohle behandelt, filtriert und eingedampft. Der Rückstand wird aus 5 ml Aceton umkristallisiert.

Ausbeute: 0,42 g (10 %), Fp. 77 - 82°C; fast farblose Kristalle, MG = 426,015.

**Beispiel 6:**

1-[3-(2-Hydroxy-3-cyclopropylmethylaminopropoxy)-4-methyl-2-thienyl]-3-phenyl-1-propanon-hydrochlorid

3,0 g Epoxid werden mit 20 ml Aminomethylcyclopropan 5 h unter Rühren am Rückfluß gekocht.

Dann wird das überschüssige Amin am Rotationsverdampfer abdestilliert. Der getrocknete Rückstand wird in wenig absolutem Toluol aufgenommen und auf einer vorbehandelten Kieselgelsäule aufgetragen. Das Kieselgel wird mit Petroläther (40/60) : Äther : Triäthylamin = 10 : 3 : 1 aufgeschlämmt, auf eine Glassinternutsche abgesaugt und mit Äther nachgewaschen und trockengesaugt. Das so basisch vorbehandelte Kieselgel wird für die chromatographische Trennung auf der Säule verwendet. Die Substanz wird mit Toluol : Äther (9 : 1) eluiert. Es werden Fraktionen zu 15 ml aufgefangen. Die so gereinigte Base wird mit ätherischer Salzsäure versetzt und in der Kälte Aceton hinzugefügt. Das Hydrochlorid kristallisiert in der Kälte aus.

Ausbeute: 0,23 g (6 %); Fp. 81 - 89°C; fast farblose Kristalle, MG = 409,972.

**Beispiel 7:** 1-[3-(2-Hydroxy-3-allylaminpropoxy)-4-methyl-2-thienyl]-3-phenyl-1-propanon-hydrochlorid

2,0 g Epoxid werden mit 15 ml Allylamin 5 h bei 49°C gerührt. Das überschüssige Amin wird am Rotationsverdampfer im Vakuum abdestilliert und der getrocknete Rückstand über eine vorbehandelte Kieselgelsäule, wie im Beispiel 6 beschrieben, gereinigt. Es wird mit Toluol : Äthanol = 9 : 1 eluiert und es werden Fraktionen zu 15 ml aufgefangen.

Ausbeute: 0,4 g (15 %); Fp. 48 - 53°C; fast farblose, stark hygroskopische Kristalle, MG = 395,945.

**Pharmakologie:**

Die erfindungsgemäßen Verbindungen wurden auf ihre antiarrhythmische Potenz und auf negativ inotrope Eigenschaften hin untersucht. Als klassische Vertreter natriumantagonistischer Antiarrhythmika ist zu erwarten, daß neben einem Schutz vor auftretenden ektopen Schlägen und Flimmern auch eine gewisse kardiodepressive Wirkung als Folge der Natriumeinstrom-Hemmung auftritt.

Dieses Grundmuster zeigen alle bislang bekannten Verbindungen der Klasse I Antiarrhythmika (nach Vaughan Williams).

**Methode:**

Für das Screening wurde der isolierte linke Meerschweinchenvorhof als Modell gewählt. Die Vorhöfe wurden durch Rechteckimpulse ( ~ 3 msec) mit einer Basisfrequenz von 2 Hz elektrisch stimuliert. Nun wurde mittels eines Extrastimulus, dessen zeitliche Verzögerung exakt variiert werden kann, die effektive Repolarisationszeit mit und ohne Verbindung bestimmt. Gleichzeitig wurde auch die Wirkung der

Verbindungen auf die Kontraktionskraft des Herzmuskels gemessen. Als Vergleichssubstanzen dienten bei allen Versuchen Chinidin und Lidocain. Um zwischen Na-antagonistischen und anderen Wirkungen unterscheiden zu können, wurden die effektive RP, die Kontraktionskraft und die Erregbarkeit des Meerschweinchenvorhofes untersucht.

Weitere Untersuchungen wurden am teilweise depolarisierten Papillarmuskel durchgeführt, um zwischen der negativ inotropen Wirkung, welche auf Blockade der schnellen Na+-Kanälchen und einer, die auf anderen Mechanismen beruht, zu unterscheiden. Im Versuch wurde die teilweise Depolarisation der Herzmuskelzellmembran durch Erhöhung der extrazellulären Kalium-Konzentration auf das ca. 4-fache erreicht.

**Ergebnisse:**

Alle getesteten Antiarrhythmika verlängerten in einer Konzentration von 10 µM die funktionelle Refraktärperiode und senkten die Kontraktionskraft. Das Verhältnis zwischen der Verlängerung der Refraktärperiode und der gleichzeitig beobachteten Kontraktionskraftsenkung war bei 1-[3-(2-Hydroxy-3-tert.pentylaminopropoxy)-4-methyl-2-thienyl]-3-phenyl-1-propanon-hydrochlorid am größten, zusätzlich konnte eine Verminderung der Erregbarkeit des Vorhofes gesehen werden. Die Verlängerung der Refraktärperiode wird dabei mit wesentlich kleineren Dosen erzielt als die Senkung der Kontraktionskraft.

Die Versuche mit den teilweise depolarisierten Membranen weisen darauf hin, daß bei den vorliegenden Verbindungen (etwa 1-[3-(2-Hydroxy-3-tert.pentylaminopropoxy)-4-methyl-2-thienyl]-3-phenyl-1-propanon-hydrochlorid) die negativ incorope Komponente nur durch den Na+-Antagonismus hervogerufen wird.

In folgender Tabelle bedeuten:

LG 84-6-07 = 1-[4-Methyl-3-(2-hydroxy-3-tert.pentyl aminopropoxy)-2-thienyl]-3-phenyl-1-propanon-hydrochlorid.

LG 84-6-08 = 1-[3-(2-Hydroxy-3-sek.butylaminopropoxy)-4-methyl-2-thienyl]-3-phenyl-1-propanon-hdrochlorid.

LG 84-6-09 = 1-[3-(2-Hydroxy-3-(2,2-dimethylpropylamino)-propoxy)-4-methyl-2-thienyl]-3-phenyl-1-propanon-hydrochlorid.

LG 84-6-11 = 1-[3-(2-Hydroxy-3-(4-methylpiperidinyl)-propoxy)-4-methyl-2-thienyl]-3-phenyl-1-propanon-hydrochlochlorid.

n = Anzahl der Tiere

| Substanz | Verlängerung der RP | | Hemmung der Kontraktionskraft | | $\frac{E_{RP}}{E_{KK}}$ |
|---|---|---|---|---|---|
| | % | n | % | n | |
| LG 84-6-07 | 66 ± 13,0 | 6 | 52 ± 4,0 | 7 | 1,3 |
| LG 84-6-08 | 129 ± 11,0 | 6 | 65 ± 4,0 | 6 | 2,0 |
| LG 84-6-09 | 112 ± 18,0 | 6 | 37 ± 4,5 | 6 | 3,0 |
| LG 84-6-11 | 157 ± 10,0 | 6 | 59 ± 3,3 | 6 | 2,7 |
| Lidocain (100 µM) | 63 ± 6,0 | 4 | 17 ± 5,0 | 4 | 3,7 |
| Chinidin (100 µM) | > 161 | 4 | 77 ± 4,0 | 4 | - |

**Patentansprüche**

1. 1-[3-(2-Hydroxy-3-alkylaminopropoxy)-2-thienyl]-3-phenyl-1-propanone der allgemeinen Formel

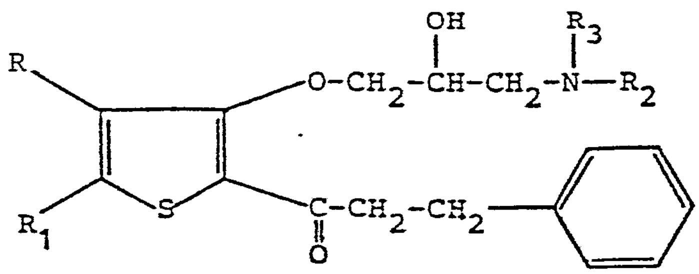

(I) ,

worin R und $R_1$ jeweils Wasserstoff oder Methyl bedeuten, wobei aber mindestens eines hievon Methyl bedeutet, $R_2$ Alkyl, Cycloalkyl oder Alkenyl mit jeweils bis zu 8 C-Atomen bedeutet und $R_3$ Wasserstoff darstellt oder $R_2$ und $R_3$ zusammen mit dem sie verbindenden Stickstoffatom einen 6-gliedrigen gesättigten heterocyclischen Ring bilden, der gegebenenfalls durch einen Alkylrest mit 1 bis 3 C-Atomen substituiert sein kann, mit der Maßgabe, daß $R_2$ eine andere Bedeutung als Propyl, n-Butyl, Isobutyl oder tert.Butyl hat, wenn $R_3$ Wasserstoff ist, und ihre Säureadditionssalze .

2. 1-[4-Methyl-3-(2-hydroxy-3-sek.butylaminopropoxy)-2-thienyl]-3-phenyl-1-propanon-chlorhydrat.

3. 1-[3-(2-Hydroxy-3-tert.pentylaminopropoxy)-4-methyl-2-thienyl]-3-phenyl-1-propanon-hydrochlorid.

4. 1-[3-(2-Hydroxy-3-(2,2-dimethylpropylamino)-propoxy)-4-methyl-2-thienyl]-3-phenyl-1-propanon-hydrochlorid.

5. 1-[3-(2-Hydroxy-3-(4-methyl-1-piperidinyl)-propoxy)-4-methyl-2-thienyl]-3-phenyl-1-propanon-hydrochlorid.

6. 1-[3-(2-Hydroxy-3-(2-methyl-1-butylamino)-propoxy)-4-methyl-2-thienyl]-3-phenyl-1-propanon-hydrochlorid.

7. 1-[3-(2-Hydroxy-3-cyclopropylmethylaminopropoxy)-4-methyl-2-thienyl]-3-phenyl-1-propanon-hydrochlorid.

8. 1-[3-(2-Hydroxy-3-allylaminopropoxy)-4-methyl-2-thienyl]-3-phenyl-1-propanon-hydrochlorid.

9. Verfahren zur Herstellung der Verbindungen der Formel (I), worin $R_1$, $R_2$ und $R_3$ die in Anspruch 1 angegebene Bedeutung haben, dadurch gekennzeichnet, daß man eine, vorzugsweise durch Umsetzung einer Verbindung der allgemeinen Formel

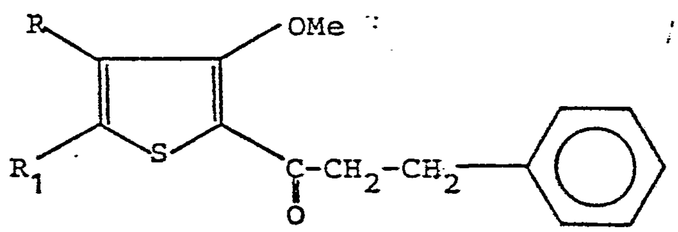

(II),

worin R und $R_1$ die oben genannte Bedeutung besitzen, und Me ein Alkalimetall-Kation bedeutet, mit Epichlorhydrin der Formel

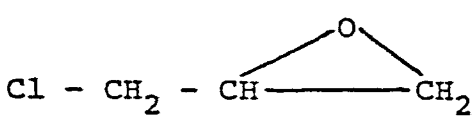

(III)

in einem reaktionsinerten Lösungsmittel oder besser in überschüssigem Epichlorhydrin bei Temperaturen zwischen 110 und 140° C erhaltene Verbindung der Formel

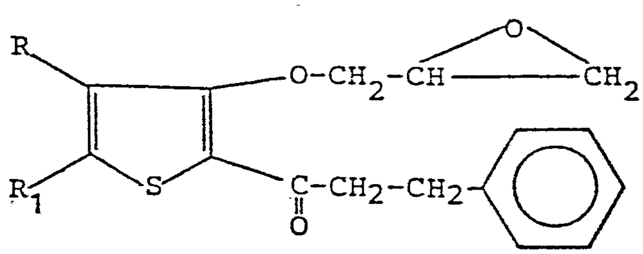

(IV) ,

worin R und $R_1$ die oben genannte Bedeutung besitzen, mit einem Alkylamin der allgemeinen Formel

$R_2R_3$ - NH (V),

worin $R_2$ und $R_3$ die oben genannte Bedeutung besitzen, in einem reaktionsinerten Lösungsmittel oder besser in überschüssigem Amin der Formel (V) bei einer Temperatur zwischen 50 und 100° C umsetzt und die erhaltenen Basen der Formel (I) gegebenenfalls in die Säureadditionssalze überführt.

10. Pharmazeutische Zusammensetzung, dadurch gekennzeichnet, daß sie eine Verbindung nach einem der Ansprüche 1 bis 8 oder ein pharmazeutisch annehmbares Säureadditionssalz hievon zusammen mit einem pharmazeutisch annehmbaren Träger, gegebenenfalls in Mischung mit anderen Wirksubstanzen, enthält.

11. Verwendung von Verbindungen der Formel (I) und deren Saäureadditionssalze zur Behandlung von Herzrhythmusstörungen.

Patentansprüch für folgende Vertragsstaat : AT

Verfahren zur Herstellung von 1-[3-(2-Hydroxy-3-alkylaminopropoxy)-2-thienyl]-3-phenyl-1 propanonen der allgemeinen Formel

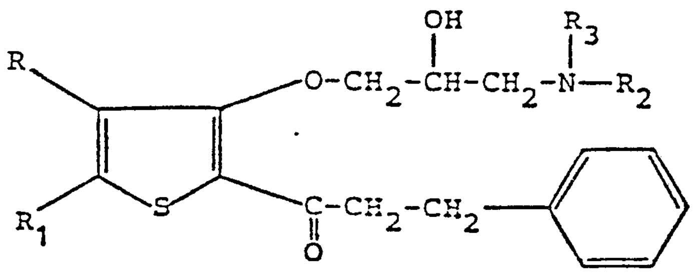

(I) ,

worin R und $R_1$ jeweils Wasserstoff oder Methyl bedeuten, wobei aber mindestens eines hievon Methyl bedeutet, $R_2$ Alkyl, Cycloalkyl oder Alkenyl mit jeweils bis zu 8 C-Atomen bedeutet und $R_3$ Wasserstoff darstellt oder $R_2$ und $R_3$ zusammen mit dem sie verbindenden Stickstoffatom einen 6-gliedrigen gesättigten heterocyclischen Ring bilden, der gegebenenfalls durch einen Alkylrest mit 1 bis 3 C-Atomen substituiert sein kann, mit der Maßgabe, daß $R_2$ eine andere Bedeutung als Propyl, n-Butyl, Isobutyl oder tert.Butyl hat, wenn $R_3$ Wasserstoff ist, und ihren Säureadditionssalzen, dadurch gekennzeichnet, daß man eine, vorzugsweise durch Umsetzung einer Verbindung der allgemeinen Formel

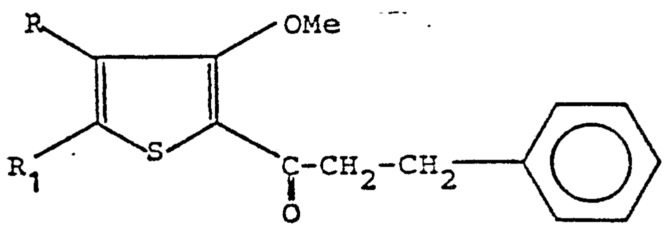

(II),

worin R und $R_1$ die oben genannte Bedeutung besitzen, und Me ein Alkalimetall-Kation bedeutet, mit Epichlorhydrin der Formel

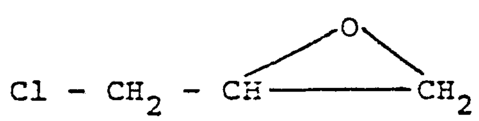

(III)

in einem reaktionsinerten Lösungsmittel oder besser in überschüssigem Epichlorhydrin bei Temperaturen zwischen 110 und 140° C erhaltene Verbindung der Formel

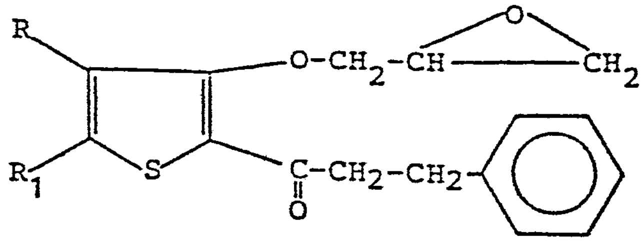

(IV) ,

worin R und $R_1$ die oben genannte Bedeutung besitzen, mit einem Alkylamin der allgemeinen Formel

$R_2R_3$ - NH (V),

worin $R_2$ und $R_3$ die oben genannte Bedeutung besitzen, in einem reaktionsinerten Lösungsmittel oder besser in überschüssigem Amin der Formel (V) bei einer Temperatur zwischen 50 und 100°C umsetzt und die erhaltenen Basen der Formel (I) gegebenenfalls in die Säureadditionssalze überführt.

Patentansprüche für folgende Vertragsstaat : ES

1. Verfahren zur Herstellung von 1-[3-(2-Hydroxy-3-alkylaminopropoxy)-2-thienyl]-3-phenyl-1propanonen der allgemeinen Formel

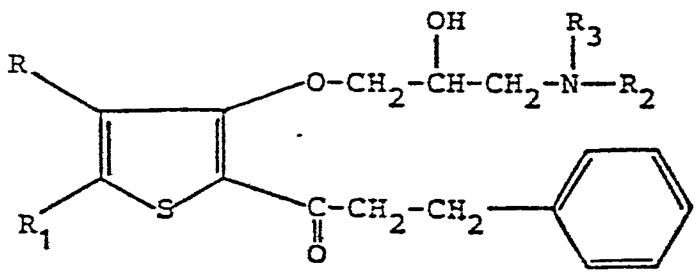

(I) ,

worin R und $R_1$ jeweils Wasserstoff oder Methyl bedeuten, wobei aber mindestens eines hievon Methyl bedeutet, $R_2$ Alkyl, Cycloalkyl oder Alkenyl mit jeweils bis zu 8 C-Atomen bedeutet und $R_3$ Wasserstoff darstellt oder $R_2$ und $R_3$ zusammen mit dem sie verbindenden Stickstoffatom einen 6-gliedrigen gesättigten heterocyclischen Ring bilden, der gegebenenfalls durch einen Alkylrest mit 1 bis 3 C-Atomen substituiert sein kann, mit der Maßgabe, daß $R_2$ eine andere Bedeutung als Propyl, n-Butyl, Isobutyl oder tert.Butyl hat, wenn $R_3$ Wasserstoff ist, und ihren Säureadditionssalzen, dadurch gekennzeichnet, daß man eine, vorzugsweise durch Umsetzung einer Verbindung der allgemeinen Formel

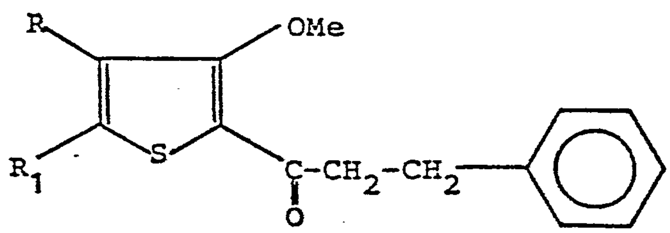

(II),

worin R und $R_1$ die oben genannte Bedeutung besitzen, und Me ein Alkalimetall-Kation bedeutet, mit Epichlorhydrin der Formel

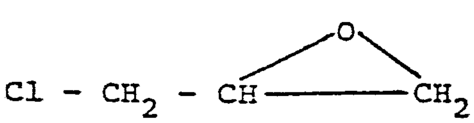

(III)

in einem reaktionsinerten Lösungsmittel oder besser in überschüssigem Epichlorhydrin bei Temperaturen zwischen 110 und 140°C erhaltene Verbindung der Formel

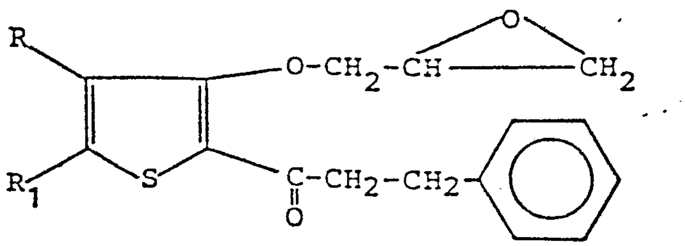

(IV) ,

worin R und $R_1$ die oben genannte Bedeutung besitzen, mit einem Alkylamin der allgemeinen Formel

$R_2R_3$ - NH (V),

worin $R_2$ und $R_3$ die oben genannte Bedeutung besitzen, in einem reaktionsinerten Lösungsmittel oder besser in überschüssigem Amin der Formel (V) bei einer Temperatur zwischen 50 und 100° C umsetzt und die erhaltenen Basen der Formel (I) gegebenenfalls in die Säureadditionssalze überführt.

2. Verwendung von Verbindungen der Formel (I) und deren Saäureadditionssalzen zur Behandlung von Herzrhythmusstörungen.

Europäisches Patentamt

**EUROPÄISCHER RECHERCHENBERICHT**

Nummer der Anmeldung

EP 87 89 0016

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| Y | EP-A-0 053 603 (LAEVOSAN-GmbH) * Ansprüche * & AT-A-369 739 (Cat. D) | 1,9-11 | C 07 D 333/32<br>A 61 K 31/38 |
| Y | DE-A-2 720 613 (BASF) * Ansprüche; Seiten 3,4 * | 1,9-11 | |
| A | DE-A-1 935 558 (HOECHST) * Seiten 8,9; Ansprüche * | 1,9-11 | |

RECHERCHIERTE SACHGEBIETE (Int. Cl.4)

C 07 D 333/00
A 61 K 31/00

Der vorliegende Recherchenbericht wurde für alle Patentansprüche erstellt.

| Recherchenort | Abschlußdatum der Recherche | Prüfer |
|---|---|---|
| DEN HAAG | 08-05-1987 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument